(19) Europäisches Patentamt
European Patent Office
Office européen des brevets

(11) Veröffentlichungsnummer: **0 264 803**
**A2**

(12) # EUROPÄISCHE PATENTANMELDUNG

(21) Anmeldenummer: **87114993.6**

(51) Int. Cl.⁴: **C07K 5/06** , //C07K1/14

(22) Anmeldetag: **14.10.87**

(30) Priorität: **20.10.86 DE 3635582**

(43) Veröffentlichungstag der Anmeldung:
**27.04.88 Patentblatt 88/17**

(84) Benannte Vertragsstaaten:
**AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmidt, Erwin, Dr.**
**Am Flachsland 26**
**D-6233 Kelkheim Taunus(DE)**
Erfinder: **Keller, Reinhold, Dr.**
**Wiesenweg 5**
**D-6232 Bad Soden am Taunus(DE)**

(54) **Verfahren zur Reinigung von N-Acyl-Aspartam.**

(57) Das α-verknüpfte Dipeptid aus Asparaginsäure und Phenylalaninester kann von der entsprechenden β-verknüpften Komponente sehr gut durch Umkristallisation des Gemisches aus gepufferter, wäßriger Lösung abgetrennt werden.

EP 0 264 803 A2

Xerox Copy Centre

## Verfahren zur Reinigung von N-Acyl-Aspartam

Für die Synthese des Süßstoffes Aspartam wird durch Umsatz von am Stickstoff geschütztem L-Asparaginsäureanhydrid mit L-Phenylalaninmethylester ein am endständigen Stickstoffatom geschütztes Aspartam erzeugt, aus dem dann die Schutzgruppe enzymatisch hydrogenolytisch oder hydrolytisch abgespalten wird.

Als Schutzgruppen eignen sich insbesondere der Benzoyl-, Phenylacetyl-, Phenoxyacetyl-, Phenoxypropionyl-, Benzyloxycarbonylrest, wie in der Deutschen Patentschrift 35 23 018 aufgeführt.

Die Umsetzung des geschützten Asparaginsäureanhydrids mit Phenylalaninester verläuft jedoch nicht einheitlich. Man erhält je nach Reaktionsbedingungen und Substituenten neben der α-Verknüpfung des Asparaginsäurerestes erhebliche Mengen des Endproduktes mit β-Verknüpfung (Chemistry and Industry, 15.7.1985, S.485).

Zur Entfernung der unerwünschten β-Komponente wird in der Deutschen Offenlegungsschrift 20 53 188 vorgeschlagen, das entstandene α/β-Gemisch erst nach der Abspaltung der Schutzgruppe beispielsweise durch Kristallisation zu reinigen. Da Aspartam aber bei höheren Temperaturen instabil ist und Zersetzungsprodukte wie Diketopiperazine bildet, (Food Technology, Juli 1984, S. 53) ist dieses Verfahren unvorteilhaft und insbesondere bei größeren Ansätzen mit starken Verlusten verbunden.

Es erschien daher vorteilhaft, die β-Komponente bereits vor Abspaltung der Schutzgruppe abzutrennen. Das Umkristallisieren aus den gebräuchlichen organischen Lösungsmitteln hat jedoch einen völlig unzureichenden Reinigungseffekt oder ist mit hohen Verlusten verbunden.

Es wurde nun überraschend gefunden, daß das α verknüpfte Dipeptid aus Asparaginsäure und Phenylalaninester von der entsprechenden β-verknüpften Komponente abgetrennt werden kann, wenn man ein Gemisch der beiden Komponenten mit Acylschutzgruppen aus wäßrigen, gepufferten Lösungen umkristallisiert, wobei eine der Komponenten in Lösung bleibt während die andere ausfällt.

Die Erfindung betrifft daher ein Verfahren zur Trennung von Gemischen von Verbindungen der allgemeinen Formeln I und II,

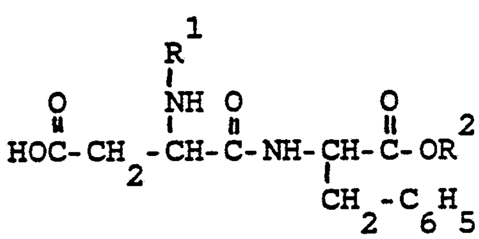

I

(α-verknüpft)

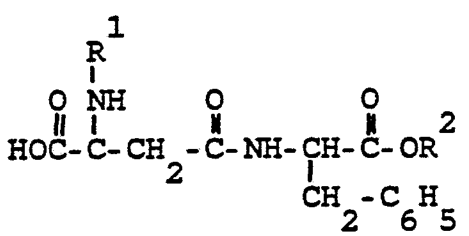

II

(β-verknüpft)

in der $R^1$ eine Aminoschutzgruppe bedeutet und $R^2$ einen Alkylrest mit 1 bis 4 Kohlenstoffatomen darstellt, das dadurch gekennzeichnet ist, daß man aus wäßrigen, gepufferten Lösungen umkristallisiert.

Obgleich das erfindungsgemäße Trennverfahren auch für Gemische weiterer N-acylierter α-and β-Aspartyl-phenylalaninester geeignet ist, wird es vorteilhaft zur Trennung der α-und β-Komponenten von N-benzoyl-, N-phenylacetyl-, N-benzyloxycarbonyl-, N-phenoxyacetyl-oder N-phenoxypropionyl-geschützten Aspartyl-phenylalaninestern eingesetzt, da diese sich besonders leicht enzymatisch, wie in der Deutschen Patentanmeldung 35 23 018 beschrieben, oder hydrogenolytisch nach der Deutschen Offenlegungsschrift 20 53 188 in das ungeschützte Aspartam überführen lassen. Weiterhin sind ebenfalls aliphatische Acylreste mit $C_1$-$C_4$ Kohlenstoffatomen, wie z.B. Formyl-, Acetyl-, Propionylkl-bzw. n-oder iso-Butyryl bevorzugt geeignet.

Besonders vorteilhaft ist die Verwendung der Phenacetyl-der Phenoxyacetyl-oder Benzyloxycarbonyl-Schutzgruppe, die sich besonders leicht einführen lassen. Bei der Einführung des Phenoxypropionylrestes erhält man entweder ein Diastereomerengemisch oder muß von enantiomereneinheitlicher Phenoxypropionsäure ausgehen.

Die Umsetzung des geschützten Asparaginsäureanhydrids kann mit Phenylalaninestern erfolgen, deren Alkoxyrest 1 bis 4 Kohlenstoffatome enthält. Geeignet sind beispielsweise n-, sio-, tert. Butyl-, n-, iso-Propyl-, Ethyl-und Methylester. Besonders vorteilhaft ist die Verwendung des Methylesters, da man auf diesem Wege ohne weitere Umesterung direkt zum Süßstoff Aspartam gelangt.

Geeignete Puffersysteme sind beispielsweise wäßrige Lösungen von hinreichend löslichen Natrium-, Kalium-, Ammonium-, Magnesium-oder Calciumsalzen der Kohlensäure, der Phosphorsäure oder wasserlöslicher Carbon-oder Phosphonsäuren. Als Ammoniumsalze sind auch die Salze organischer primärer, sekundärer und tertiärer Amine sowie quartäre Ammoniumsalze oder Salze von Guanidin , Biguanid oder Thioharnstoff geeignet.

Die Puffersysteme können durch Auflösen der geeigneten Salze einzeln oder in Mischung sowie durch Auflösen der freien Basen und Säuren hergestellt werden.

Die Puffersysteme können im Überschuß, in stöchiometrischen Mengen oder im Unterschuß zugesetzt werden. Man kann einen beliebigen Überschuß des Puffersystems einsetzen. Aus wirtschaftlichen Gründen und zur Gewinnung von salzfreiem α-Aspartam bringt jedoch ein Überschuß von mehr als 2 Mol Puffer keinen Vorteil.

Er ist besonders vorteilhaft, wenn man beispielsweise in kurzen Vorversuchen die Menge des Puffers ermittelt, die gerade ausreicht, das vorhandene β-Alkylaspartam in Lösung zu halten. Die optimale Menge sowie der optimale pH-Wert muß im Einzelfall ermittelt werden. Er hängt weitgehend von der Löslichkeit der α-und der β-Komponente und deren Salzen sowie von deren Hydrolysekonstanten ab. Der pH-Wert kann auch dadurch eingestellt werden, daß man der Lösung oder Suspension des Acylaspartam die geeignete Menge einer Base, beispielsweise Natronlage, wäßriges Ammoniak oder ein Amin zusetzt. In solchen Fällen wirkt das Acylaspartam selbst als Pufferkomponente. Der Zusatz freier Alkalibasen führt jedoch leicht, insbesondere bei höheren Temperaturen zur Verseifung der Alkylestergruppe des Aspartams.

Der pH-Wert der wäßrigen Lösung kann sich während des erfindungsgemäßen Prozesses ändern. Verwendet man beispielsweise Natronlauge im Unterschuß, so geht man von einem pH-Wert von 14 aus, der nach Zusatz des Acylaspartam auf 4 zurückgeht. Wie bereits angegeben tritt dabei leicht im alkalischen Bereich eine teilsweise Verseifung der Methylestergruppen ein. Andererseits ist der Reinigungseffekt bereits wesentlich geringer, wenn man von einer Phosphatpufferlösung von pH 3 vor Zusatz des Alkylaspartam ausgeht. Eine optimale Trennung der α-und β-Komponente erhält man in Systemen, deren pH-Wert nach Zugabe des Acylaspartam bei 4 bis 6 liegt. Eine obere Begrenzung des pH-Wertes der Pufferlösung vor Zusatz des Acylaspartam ist nicht möglich, da beispielsweise eine wäßrige Kaliumcarbonatlösung bereits beim Erwärmen oder beim Durchleiten von Inertgas unter Abspaltung von Kohlendioxid Kaliumhydroxid bildet. (Gmelin, System Nr. 22, K 1936-38, S. 822). Vorteilhaft leigt der pH-Wert der Pufferlösung vor Zusatz des Acylaspartam zwischen 5 und 12, insbesondere, um Verluste an Methylester zu vermeiden.

Als Puffersysteme besonders geeignet sind beispielsweise:

Ammonium-oder Alkaliphosphatpuffer: pH 4 bis 7
Ammonium-oder Alkalicitratpuffer: pH 6,5
Ammonium-oder Alkaliboratpuffer: pH 6,0
Alkalihydrogencarbonatlösung : ph 8
Ammonium-oder Alkalicarbonatlösung : pH 12
Ammoniumhydrogencarbonatlösung : pH 7

Besonders bevorzugt ist der physiologisch unbedenkliche und preiswerte Phosphatpuffer, mit dessen Hilfe der pH-Wert in breiten Konzentrationsbereichen beliebig einzustellen ist.

Die Pufferlösungen werden in der üblichen Weise hergestellt, wie z.B. in Küster/Thiel/Fischbeck, logarithmische Rechentafeln, 100. Auflage, Berlin 1969, S. 263 ff. angegeben ist.

Das Acylaspartam wird aus wäßriger Lösung umkristallisiert. Dabei kann es je nach Reaktionsbedingungen als freie Verbindung oder als anionischer Bestandteil eines Salzes anfallen. Als solches kann es direkt für die weiteren Stufen der Aspartamsynthese eingesetzt werden. Man kann aber auch das freie Acylaspartam mit Hilfe von Säuren freisetzen. Es kann dann in fester Form oder durch Extrahieren mit Hilfe von Lösungsmitteln isoliert werden.

Zur Reinigung ist es nicht erforderlich, daß das Acylaspartam beim Erhitzen des Puffersystems vollständig in Lösung geht. Vielmehr trennen sich α-und β-Komponente auch dann, wenn unter geeigneten Bedingungen ein Teil des Substanzgemisches nicht in Lösung geht oder nur schmilzt.

Die Kristallisation kann durch Zusatz wassermischbarer Lösungsmittel im Einzelfall beeinflußt werden. Man kann auch das Reinigungsprodukt durch Zusatz mit Wasser begrenzt mischbarer Lösungsmittel vor, während oder nach dem Umkristallisieren in gelöster Form isolieren.

Im allgemeinen genügt ein einmaliger Umkristallisierungsprozeß um weit über 90 %-iges α-Acylaspartam zu erhalten. Bei Gemischen mit sehr hohen β-Anteil (30 - 50 %) ist es unter Umständen vorteilhaft, den Reinigungsprozeß ein-oder mehrmals zu wiederholen.

Beispiel

Die Prozeßbedingungen und Ergebnisse zeigt die folgende Tabelle.

Je 10 g des 99 %-igen α/β-Dipeptidgemischs werden in den angegebenen Lösungsmitteln einige Minuten erhitzt, wobei das Rohprodukt entweder vollständig in Lösung ging (ja) oder nicht (nein). Anschließend wurde auf Raumtemperatur abgekühlt, abgesaugt, mit wenig Wasser nachgewaschen und bei 60° getrocknet.

Analytik:

Der α-und β-Gehalt der Produkte wurde mittels HPLC bestimmt.
Gerät: Spark Holland Modell SpH 126.
Säule: VA Stahl 4,6 mm Nennwert, 25 cm Länge
UV-Detektor: 254 nm.
Registrierung: Computing-Integrator
Arbeitsweise: Isokratisch bei 40° im Säulenofen.
für Phenylacetylaspartam: Mobile Phase: Acetonitril mit 2 ml 85 %-iger $H_3PO_4$/1; 70 bar; Strömung 1 ml/min; Dosierung 20 ml.
Retentionszeit der α-Komponente: 5,1 min.
Retentionszeit der β-Komponente: 6,1 min.
für Benzoxycarbonylaspartam: Mobile Phase: 60 % Acetonitril mit 40 % 0,01 molarer wäßriger $NaClO_4$-Lösung; 30 bar; Strömung 1,5 ml/min; Dosierung 20 ml.
Retentionszeit der α-Komponente: 4,7 min.
Retentionszeit der β-Komponente: 8,9 min.

**Tabelle**

Einwaage: je 10,0 g Rohprodukt; $R^2 = CH_3$

| Rohprodukt $R^1$ | Rohprodukt α-Gehalt % | Lösungsmittel | vollständig gelöst | Reinigungsprodukt α-Gehalt % | Reinigungsprodukt α-Ausbeute % | pH-Wert vor Lösen der Substanz | pH-Wert nach Lösen der Substanz |
|---|---|---|---|---|---|---|---|
| 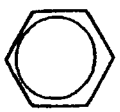 $-CH_2-$ | 81,3 | 100 ml 0,4 molare Na-phosphatpuffer-Lsg. | ja | 99,5 | 98 | 6,5 | 5 |
| | | 100 ml 0,25 molare $NaHCO_3$-Lsg | nein | 95 | 45 | 8 | 5 |
| | | 100 ml 0,07 molare $NaHCO_3$-Lsg | nein | 95 | 97 | 8 | 4 |
| | | 100 ml 0,025 molare $K_2CO_3$-Lsg | nein | 99 | 75 | 12 | 4 |
| | | 100 ml 0,5 molare $NH_3$-Lsg | ja | 100 | 27 | 12 | 6 |
| | | 100 ml 0,07 molare $NH_3$-Lsg | nein | 90 | 62 | 12 | 4 |
| 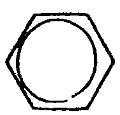 $-CH_2-O-$ | 83,8 | 100 ml 0,45 molare K-Phosphatpuffer-Lsg | nein | 85 | 92 | 5 | 4 |
| | | | nein | 92 | 92 | 6 | 5 |
| | | | ja | 99 | 93 | 7 | 5 |
| | | 110 ml 0,45 molare Na-Citratpuffer-Lsg | ja | 91 | 99 | 4,5 | 5 |

## Ansprüche

1. Verfahren zur Trennung von Gemischen von Verbindungen der allgemeinen Formeln I und II,

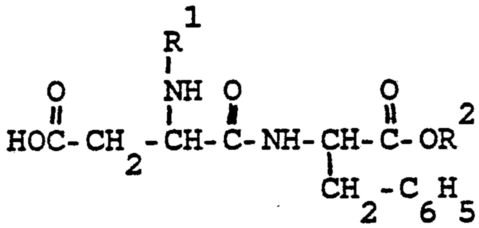

I (α-verknüpft)

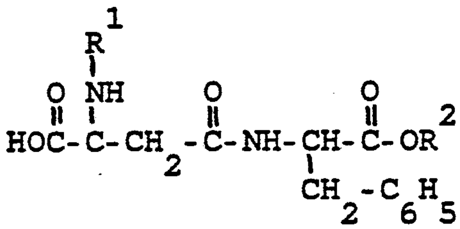

II (β-verknüpft)

in der $R^1$ eine Aminoschutzgruppe bedeutet und $R^2$ einen Alkylrest mit 1 bis 4 Kohlenstoffatomen darstellt, dadurch gekennzeichnet, daß man aus wäßrigen, gepufferten Lösungen umkristallisiert.

2. Verfahren nach Anspruch 1, dadurch gekennzeichnet, daß $R^1$ eine Benzoyl-, Phenylacetyl-, Phenoxyacetyl-, Phenoxypropionyl-Benzyloxycarbonylgruppe oder einen aliphatischen Acylrest mit 1 bis 4 Kohlenstoffatomen bedeutet.

3. Verfahren nach Anspruch 1 oder 2, dadurch gekennzeichnet, daß $R^2$ eine Methylgruppe bedeutet.

4. Verfahren nach einem oder mehreren der Ansprüche 1 bis 3, dadurch gekennzeichnet, daß man eine gepufferte Lösung mit einem pH-Wert von 3 bis 14 verwendet.

5. Verfahren nach Anspruch 4, dadurch gekennzeichnet, daß man eine gepufferte Lösung mit einem pH-Wert von 5 bis 12 verwendet.

6. Verfahren nach einem oder mehreren der Ansprüche 1 bis 5, dadurch gekennzeichnet, daß man die Lösung von Ammonium-und/oder Alkaliphosphat, -citrat, -borat, -hydrogencarbonat oder -carbonat als Puffer verwendet.

7. Verfahren nach Anspruch 6, dadurch gekennzeichnet, daß man Ammonium-und/oder Alkaliphosphatpuffer verwendet.